# EUROPEAN PATENT SPECIFICATION

(11) **EP 0 527 941 B1**
(45) Date of publication and mention of the grant of the patent: **18.09.1996**
(21) Application number: 91910623.7
(22) Date of filing: 15.04.1991
(51) Int. Cl.: C12Q 1/68

(54) **IDENTIFICATION OF NOVEL DRUGS AND REAGENTS**
IDENTIFIKATION VON NEUEN MEDIKAMENTEN UND REAGENZIEN
PROCEDES D'IDENTIFICATION DES NOUVEAUX MEDICAMENTS ET REACTIFS

(30) Priority: 01.05.1990 US 517240
(43) Date of publication of application: 24.02.1993
(73) Proprietor: ISIS PHARMACEUTICALS, INC., Carlsbad, CA 92008 (US)
(72) Inventor: MIRABELLI, Christopher, K., Encinitas, CA 92024 (US); VICKERS, Timothy, Vista, CA 92084 (US); ECKER, David, J., Carlsbad, CA 92009 (US)
(74) Representative: Hallybone, Huw George
(86) International application number: US9102574
(87) International publication number: WO9117266

(56) References cited:
- TRENDS IN GENETICS vol. 1, no. 1 , January 1985 , ELSEVIER SCIENCE PUBLISHERS, AMSTERDAM,NL; pages 22 - 25 H. WEINTRAUB ET AL. 'Anti-sense RNA as a molecular tool for genetic analysis'
- GENE vol. 44, no. 2,3 , 1986 , ELSEVIER PUBLISHERS, N.Y., U.S.; pages 177 - 183 A.R. OLIPHANT ET AL. 'Cloning of random-sequenced oligonucleotides'
- PHARMACEUTICAL RESEARCH, vol. 5, no. 9, 1988; ZON, pp. 539-549/
- G.M. HASLER et al., "A Rapid Quantitative Bioassay Based on the Human Immunodeficiency Virus Trans-Activator", Mary Ann Liebert Inc. (publ.); pp. 507-516/
- CANCER RESEARCH, vol. 48, 15 May 1988; STEIN et al., pp. 2659-2668/
- ANALYTICAL BIOCHEMISTRY, vol. 172, 1988; MARCUS-KEKURA, pp. 289-295/

## Description

### FIELD OF THE INVENTION

This invention relates to the identification, preparation and use of novel therapeutic, diagnostic and research compositions and to methods for their use. More particularly, this invention relates to novel antisense compositions, compositions which can interact with nucleic acids by hybridization to effect their function and to cause changes in biological activity thereby. Methods for identifying such antisense compositions are provided which lead to novel therapeutic, diagnostic and research compositions and to methods for diagnosis, treatment and experimentation.

### BACKGROUND OF THE INVENTION

It is well known that most of the bodily states in mammals including most disease states, are effected by proteins. Such proteins, either acting directly or through their enzymatic functions, contribute in major proportion to many diseases in animals and man. Classical therapeutics has generally focused upon interactions with such proteins in efforts to moderate their disease causing or disease potentiating functions. Recently, however, attempts have been made to moderate the actual production of such proteins by interactions with molecules that direct their synthesis, intracellular RNA. By interfering with the production of proteins, it has been hoped to effect therapeutic results with maximum effect and minimal side effects. It is the general object of such therapeutic approaches to interfere with or otherwise modulate gene expression leading to undesired protein formation.

One method for inhibiting specific gene expression which has been adopted to some degree is the "antisense" approach. A number of workers have reported such attempts. Pertinent reviews include C.A. Stein & J.S. Cohen, *Cancer Research,* vol. 48, pp. 2659-2668 (1988); J. Walder, *Genes* & *Development,* vol. 2, pp. 502-504 (1988); C.J. Marcus-Sekura, *Anal. Biochemistry,* vol. 172, 289-295 (1988); G. Zon, *Journal of Protein Chemistry,* vol. 6, pp-131-145 (1987); G. Zon, *Pharmaceutical Research,* vol. 5, pp. 539-549 (1988); A. R. Van der Krol, J.N. Mol, & A.R. Stuitje, *BioTechniques,* vol. 6, pp. 958-973 (1988) and D.S. Loose-Mitchell, *TIPS,* vol. 9, pp. 45-47 (1988). Each of the foregoing provide background concerning general antisense theory and prior techniques.

Prior attempts at antisense therapy have provided oligonucleotides or oligonucleotide analogues which are designed to bind in a specific fashion to--which are specifically hybridizable with-- a specific mRNA by hybridization. Such analogues are intended to inhibit the activity of the selected mRNA-- to interfere with translation reactions by which proteins coded by the mRNA are produced- by any of a number of mechanisms. The inhibition of the formation of the specific proteins which are coded for by the mRNA sequences interfered with have been hoped to lead to therapeutic benefits.

A number of chemical modifications have been introduced into antisense oligonucleotides to increase their therapeutic activity. Such modifications are designed to increase cell penetration of the antisense oligonucleotides, to stabilize them from nucleases and other enzymes that degrade or interfere with the structure or activity of the oligonucleotide analogues in the body, and to improve their pharmacokinetic properties. At present, however, no generalized antisense oligonucleotide therapeutic or diagnostic scheme has been found. Prior efforts have been limited by the inability to deliver a sufficient quantity of antisense oligonucleotide into appropriate cells for effective activity to take place. The activity of the antisense oligonucleotides presently available has not been sufficient for effective therapeutic, research reagent, or diagnostic use in any practical sense. Accordingly, there has been and continues to be a long-felt need for oligonucleotides and oligonucleotide analogues which are capable of effective therapeutic and diagnostic antisense use.

This long-felt need has not been satisfied by prior work in the field of antisense oligonucleotide therapy and diagnostics. Others have failed to provide materials which are, at once, therapeutically or diagnostically effective at reasonable rates of application. In particular, there has been no generalized methodology for identifying oligonucleotides which are capable of effectively treating infectious agents. All known, prior methodologies for identifying such nucleotides have required prior knowledge of the nucleic acid sequence for that portion of nucleic for which hybridization is desired. It is therefore greatly to be desired to identify effective antisense oligonucleotides for infectious agents, which identification does not require such prior knowledge of the nucleic acid sequence.

### OBJECTS OF THE INVENTION

It is an object of this invention to provide methods for identifying oligonucleotides useful in antisense therapy of diseased states.

A further object is to identify oligonucleotides useful for the determination of the status of bodily functions of animals.

Yet another object is to identify oligonucleotides which are useful as research reagents such as for blocking gene expression of particular RNA molecules.

An additional object of the invention is to identify loci of nucleic acids which control the expression of polypeptides having a significant effect on a bodily function of an animal.

A further object is to identify and provide such oligonucleotides without the need for prior knowledge of the sequence of nucleic acid for which hybridization is desired.

Other objects will become apparent to persons of ordinary skill in the art from a review of the present specification.

### SUMMARY OF THE INVENTION

Although antisense oligonucleotides are currently being investigated as potential therapeutic agents, it is not clear how to select the best possible nucleotide sequence to inhibit the expression of a particular mRNA. Moreover, in many cases it is even difficult to decide what gene to target for antisense inhibition to alter the course of a disease. The method of the present invention overcomes these difficulties.

In accordance with this invention, methods are provided for identifying oligonucleotides capable of hybridizing with nucleic acid of an infectious agent comprising providing a plurality of vectors which include substantially randomly sequenced oligonucleotides and incorporating those vectors into cells. The cells are infected with the infectious agent and provided with conditions for growth of the cells. Cells which are resistant to the infection are then identified. Preferably, the nucleic acid sequence of the included oligonucleotide is determined for those cells resistant to the infection.

In accordance with preferred embodiments, the substantially randomly sequenced oligonucleotides are prepared through solid state synthesis or otherwise and comprise from about 10 to about 100 nucleic acid subunits.

The cells used are preferably selected to be easily infectable by the infectious agent.

In accordance with other embodiments, reagents are prepared having as at least a component thereof the nucleic acid sequence of the inserted oligonucleotide which has been identified as coming from a cell resistant to the infectious agent. D1 and D2 describe the screening of cDNA libraries to identify unknown genes by a "subtractive" hybridisation technique whereby antisense forms of the cDNA library made from cellular mRNA are reintroduced into the cell from which the library was made and phenotypic alterations determined.

### BRIEF DESCRIPTION OF THE DRAWINGS

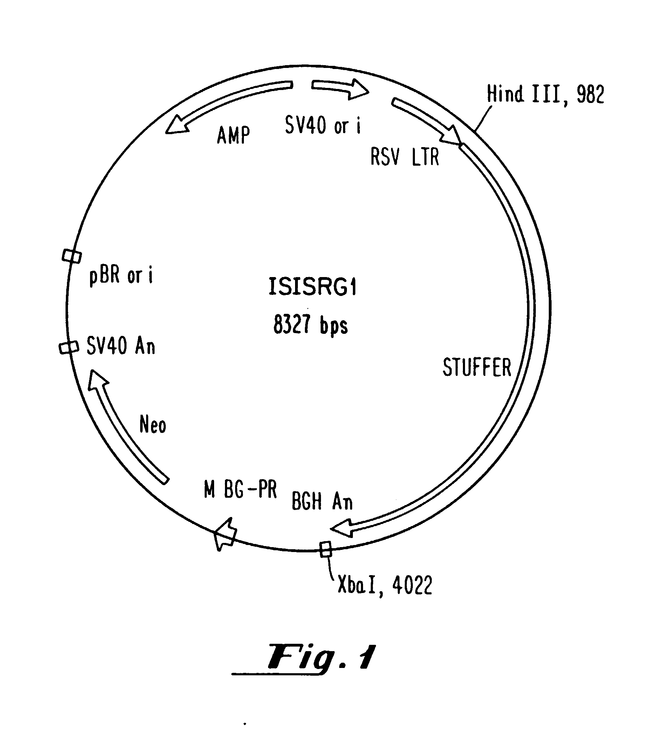

Figure 1 depicts a schematic structure of the vector ISIS RG-1.

### DETAILED DESCRIPTION OF THE INVENTION

The present invention provides a method of identifying anti-sense oligonucleotide sequences that inhibit gene expression of an infectious or other agent. Expression vectors containing oligonucleotide sequences formed by random synthesis are transformed into cells. The cells are then infected with the infectious or other agent. Oligonucleotides that inhibit gene expression of the infectious agent and which are thus useful in suppressing the effects of the agent are then selected by selecting cells that do not exhibit effects of infection with the infectious agent; for example, the cells do not die, the cells grow at an increased rate, or exhibit other behavior suggestive of modulation of the infection. Once such cells have been identified, the sequence of the oligonucleotide having the ameliorative effect is identified. Identification may be accomplished by recovering the vector and sequencing the region containing the inserted nucleic acid material.

The cells, expression vectors and method of selecting for the oligonucleotide sequence will be determined by the type of infectious or other agent. For example, if it is desired to identify anti-sense oligonucleotides to a herpesvirus, the cell type chosen will be one that can be infected with the particular herpesvirus, and the expression vector will be one that is compatible with the cell chosen.

In accordance with this invention, the term "random" as applied to sequences of nucleic acid comprehends truly random oligonucleotides formed through solid state synthesis. Even this protocol need not be completely and statistically random in fact, however. Thus, enrichment of oligonucleotides in certain bases may be desired in accordance with some embodiments of this invention.

The oligonucleotide fragments of portions are generally of a size as to be effective in the performance of this invention. Generally, from about 20 to 100 bases are employed for the synthetically generated random sequences.

The manipulation of expression vectors and oligonucleotides and the transformation of cells is performed according to standard techniques such as may be found in Maniatis et al., Molecular Cloning, A Laboratory Manual, Cold Spring Harbor Press, Cold Spring Harbor, New York, 1982.

Expression vectors suitable for use in the methods of the invention include commercially available plasmid vectors such as pMAMM-NEO (Clonetech) and other expression vectors. It is preferable that the plasmid expression vector contain at least one gene to allow construction and recovery of the vector, such as an antibiotic resistance gene such as the ampicillin resistance gene. It is also preferable that the plasmid expression vector contain an inducible promoter and transcriptional initiations region to express the random sequences and a polyadenylation signal to stabilize the random sequences. This may or may not include an intron for more efficient expression.

A preferred plasmid expression vector is ISIS RG-1 which is illustrated schematically in figure 1. This vector contains the neomycin gene for G418 selection of stably transformed cells in culture and the ampicillin gene for bacterial amplification. Inserts can be directionally cloned by cutting at the Hind III and Xba I sites, releasing a stuffer fragment. The Hind III site is immediately 3' to the RSV promoter and the xba I site is immediately 5' of the bovine growth hormone (BGH) poly (A) site.

Random sequences can be prepared by use of a DNa synthesizer to generate a length of DNA with an equimolar mixture of A, G, C, T at each position. The complimentary strand can be generated using a DNA primer and DNA polymerase. By making DNA ends of the random sequence complementary to the overhangs left by restriction enzyme cleavage it is possible to clone the random mixture into the expression vector.

Once expression vectors containing oligonucleotides are constructed, the expression vectors are inserted into cells susceptible of infection with the infectious agent. The cells may already be infected with the infectious agent or they may be infected with the infectious agent after insertion of the expression vector. The expression vectors are inserted into the cells by standard methods, such as calcium phosphate transfection or electroporation.

The cells are then cultured and oligonucleotides that are anti-sense to mRNA of the infectious agent are selected. Selection methods will depend on the type of cell and the effects of the infectious agent. For example, neommyosin resistant cells can be propagated to confluency in 10 cm tissue culture dishes. When confluency is reached the entire population is infected with HSV-1 at a low MOI. Media is changed at frequent intervals following the infection to reduce the frequency of secondary infection. Cells which survive the infection are grown to confluency, then infected a second time in the same manner. This process is repeated until all cells survive an infection or until individual resistant colonies are selected.

Once cells containing the desired oligonucleotides have been selected, the sequences of the oligonucleotides having activity are determined. Identification of the oligonucleotide sequence may be accomplished by several methods. The oligonucleotide may be removed from the expression vector and sequenced. Alternatively, the oligonucleotide sequence may be amplified in the cell by polymerase chain reaction, using appropriate primers, and the sequence of the amplified sequence determined.

### Example

### Vector Construction

The following method describes a scheme to generate random-synthetic oligomers of defined length, to screen for sequences to specifically inhibit HSV infection of mammalian cells in culture. The vector used will be ISIS RG-1. This vector contains the neomycin gene for G418 selection of stably transformed cells in culture and the ampicillin gene for bacterial amplification. Inserts can be directionally cloned by cutting at the Hind III and xba I sites, releasing a stuffer fragment. The Hind III site is immediately 3' to the RSV promoter and the Xba I site is immediately 5' of the bovine growth hormone (BGH) poly (A) site. (see fig. 1)

A random population of oligonucleotides will be produced such that the 5' end of each oligomer consists of the Hind III site, d(CAAGCTTG). This is followed by approximately 25 nt of random sequence, then the tail sequence d(TCTAGAGAAAAA), creating an Xba I site and poly A tail. A primer complementary to the 3' end sequence, 5'd(TTTTTCTCTAGA)3', will then be used as a primer for the synthesis of complementary strands to each of the random oligomers produced creating double strand molecules. The population of oligomers will then be subject digestion with Hind III and xba I to give cohesive ends compatible with the vector.

Transformation/selection Once the efficiency of the constructions has been determined, the plasmids will be transfected as a population into CV-1 or HeLa cells using the method of Chen and Okayama. Stable transformants will be selected on the basis of resistance to G418. The population of stably transformed cells will then be infected with HSV. Surviving colonies presumably will be expressing mRNA which is interacting to specifically block HSV infection. The plasmid DNA responsible for the effect can then be isolated from the transformed cells by PCR using Hind III and xba I primers.

## Claims

1. A method for identifying an oligonucleotide capable of hybridizing with nucleic acid of an infectious agent comprising:
(a) providing a plurality of vectors which include substantially randomly sequenced synthetic oligonucleotides;
(b) incorporating the vectors into cells;
(c) infecting the cells with the infectious agent;
(d) providing conditions for growth of the cells;
(e) identifying cells which are resistant to the infection; and
(f) determining the nucleic acid sequence of the included oligonucleotide in a resistant cell.

2. The method of claim 1 wherein the oligonucleotides included in the vectors comprise from about 10 to about 100 nucleic acid subunits and are prepared through a solid state synthetic technique.

3. The method of claim 1 wherein the cells are selected to be infectable by the infectious agent.

4. The method of claim 1 wherein the infectious agent is a herpesvirus and the cells are epithelial cells.

5. A process for the preparation of oligonucleotide capable of hybridizing with nucleic acid of an infectious agent comprising the steps of identifying an oligonucleotide included in a resistant cell using the method of claims 1 to 4 and preparing an oligonucleotide comprising the said included oligonucleotide.

6. A process for the preparation of a pharmaceutical composition for the treatment of an infectious agent comprising the steps of identifying an oligonucleotide included in a resistant cell using the method of claims 1 to 4, preparing an oligonucleotide comprising the said included oligonucleotide and admixing with a pharmacologically-acceptable carrier.

## Patentansprüche

1. Eine Methode zur Identifizierung eines Oligonukleotids, das zur Hybridisierung mit Nukleinsäure eines infektiösen Mittels befähigt ist, welche Methode umfaßt:
(a) Bereitstellung einer Vielzahl von Vektoren, die synthetische Oligonukleotide mit im wesentlichen regelloser Sequenz enthalten;
(b) Einbau der Vektoren in Zellen;
(c) Infizierung der Zellen mit dem infektiösen Mittel;
(d) Schaffung von Bedingungen für das Zellwachstum;
(e) Identifizierung der Zellen, die gegen die Infektion resistent sind; und
(f) Bestimmung der Nukleinsäuresequenz des enthaltenen Oligonukleotids in einer resistenten Zelle.

2. Die Methode nach Anspruch 1, worin die Oligonukleotide, die in den Vektoren enthalten sind, etwa 10 bis etwa 100 Nukleinsäure-Untereinheiten umfassen und über ein Festkörper-Syntheseverfahren hergestellt sind.

3. Die Methode nach Anspruch 1, worin die Zellen so ausgewählt werden, daß sie durch das infektiöse Mittel infizierbar sind.

4. Die Methode nach Anspruch 1, worin das infektiöse Mittel ein Herpes-Virus ist und die Zellen Epithelialzellen sind.

5. Ein Verfahren zur Herstellung eines Oligonukleotids, das zur Hybridisierung mit einer Nukleinsäure eines infektiösen Mittels befähigt ist, welches Verfahren umfaßt: die Stufen der Identifizierung eines Oligonukleotids, das in einer resistenten Zelle enthalten ist, unter Einsatz der Methode nach den Ansprüchen 1 bis 4 und der Herstellung eines Oligonukleotids, das das genannte enthaltene Oligonukleotid umfaßt.

6. Ein Verfahren zur Herstellung einer pharmazeutischen Zusammensetzung zur Behandlung eines infektiösen Mittels, welches Verfahren umfaßt: die Stufen der Identifizierung eines in einer resistenten Zelle enthaltenen Oligonukleotids unter Einsatz der Methode nach den Ansprüchen 1 bis 4, der Herstellung eines das genannte enthaltene Oligonukleotid umfassenden Oligonukleotids und des Mischens mit einem pharmakologisch verwendbaren Träger.

## Revendications

1. Procédé d'identification d'un oligonucléotide capable de s'hybrider avec l'acide nucléique d'un agent infectieux, comprenant les étapes qui consistent à:
(a) fournir une multitude de vecteurs qui comprennent des oligonucléotides synthétiques séquencés essentiellement au hasard;
(b) incorporer les vecteurs dans des cellules;
(c) infecter les cellules avec l'agent infectieux;
(d) fournir les conditions pour assurer la croissance des cellules;
(e) identifier les cellules qui sont résistantes à l'infection; et
(f) déterminer la séquence d'acides nucléiques de l'oligonucléotide incorporé dans une cellule résistante.

2. Procédé selon la revendication 1, dans lequel les oligonucléotides incorporés dans les vecteurs renferment environ 10 à environ 100 sous-unités d'acide nucléique et sont préparés grâce à une technique de synthèse à l'état solide.

3. Procédé selon la revendication 1, dans lequel les cellules sont sélectionnées pour la capacité de l'agent infectieux à les infecter.

4. Procédé selon la revendication 1, dans lequel l'agent infectieux est un virus de l'herpès et les cellules sont des cellules épithéliales.

5. Procédé pour la préparation d'un oligonucléotide capable de s'hybrider avec l'acide nucléique d'un agent infectieux, comprenant les étapes qui consistent à identifier un oligonucléotide incorporé dans une cellule résistante à l'aide du procédé selon les revendications 1 à 4 et à préparer un oligonucléotide comprenant ledit oligonucléotide incorporé.

6. Procédé pour la préparation d'une composition pharmaceutique destinée au traitement d'un agent infectieux, comprenant les étapes qui consistent à identifier un oligonucléotide incorporé dans une cellule résistante à l'aide du procédé selon les revendications 1 à 4, à préparer un oligonucléotide comprenant ledit oligonucléotide incorporé et à le mélanger avec un véhicule acceptable d'un point de vue pharmacologique.
